Europäisches Patentamt

European Patent Office

Office européen des brevets

(11) Publication number: **0 331 186 B1**

# EUROPEAN PATENT SPECIFICATION

(45) Date of publication of patent specification: **10.08.94**

(51) Int. Cl.5: **C12P 21/08**, C12P 21/02, G01N 33/68, G01N 33/543

(21) Application number: **89103765.7**

(22) Date of filing: **03.03.89**

(54) **Monoclonal antibody selectively binding to novel g-csf derivative.**

(30) Priority: **04.03.88 JP 51357/88**

(43) Date of publication of application:
**06.09.89 Bulletin 89/36**

(45) Publication of the grant of the patent:
**10.08.94 Bulletin 94/32**

(84) Designated Contracting States:
**DE FR GB**

(56) References cited:
**EP-A- 0 225 583**
**WO-A-87/01132**

**NATURE, vol. 319, 30th January 1986, pages 415-418, London, GB; S. NAGATA etal.: "Molecular cloning and expression of cDNA for human granulocyte colony-stimulating factor"**

(73) Proprietor: **KYOWA HAKKO KOGYO CO., LTD.**
**6-1, Ohtemachi 1-chome**
**Chiyoda-ku Tokyo 100(JP)**

(72) Inventor: **Yoshida, Hajime**
**9-18, Isobe**
**Sagamihara-shi Kanagawa(JP)**

(74) Representative: **Kinzebach, Werner, Dr. et al**
**Patentanwälte**
**Reitstötter, Kinzebach und Partner**
**Postfach 86 06 49**
**D-81633 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

FIELD OF THE INVENTION

This invention relates to a monoclonal antibody against ND28, a derivative of human granulocyte colony stimulating factor (hereinafter abbreviated as G-CSF), which is useful for the purification and quantitative analysis of ND28.

BACKGROUND OF THE INVENTION

A monoclonal antibody against natural G-CSF is disclosed in JP-A-63-180860 (the term "JP-A" as used herein means "an unexamined published Japanese patent application".), but no monoclonal antibody against ND28, a derivative of G-CSF, is so far known.

ND28 differs from natural G-CSF in that the 1st, 3rd, 4th, 5th and 17th amino acids in the latter, threonine (Thr), leucine (Leu), glycine (Gly), proline (Pro) and cysteine (Cys), have been replaced by alanine (Ala), threonine (Thr), tyrosine (Tyr), arginine (Arg) and serine (Ser), respectively. ND28 is disclosed in EP-A-0272703.

EP-A-0 225 583 discloses a monoclonal anti-human granulocyte colony stimulating factor antibody.

SUMMARY OF THE INVENTION

As a result of advancements in DNA recombination techniques, it is now possible to produce ND28 in large quantities using a microorganism such as Escherichia coli.

Because of this, and due to the demand for ND28 as a therapeutic agent in pure form , there has been a need for an excellent purification method for ND28 and a simple method for quantitatively analyzing the same at a high degree of accuracy. Further, it has been desired to develop an anti-ND28 monoclonal antibody selectively binding to ND28 and not cross-reactive with natural G-CSF.

An object of this invention is to provide an anti-ND28 monoclonal antibody, a method of efficiently purifying ND28 using the same and a method of analyzing ND28 simply, rapidly, specifically and a high accuracy using the same.

As a result of studies to obtain a monoclonal antibody that can be used for the purification and quantitative analysis of ND28, we have found that the hybridoma, formed by the ordinary cell fusion technique from mouse myeloma cells and the splenic cells of a mouse immunized with ND28, a derivative of G-CSF having the amino acid sequence as shown in Table 1, is capable of producing a monoclonal antibody that specifically reacts with ND28 and hence can be used for the purification and quantitative analysis of the same. This invention was accomplished on the basis of these findings.

## Table 1

```
Ala Pro Thr Tyr Arg Ala Ser Ser Leu Pro Gln Ser Phe Leu Leu Lys Ser Leu Glu Gln
                                    10                                  20

Val Arg Lys Ile Gln Gly Asp Gly Ala Ala Leu Gln Glu Lys Leu Cys Ala Thr Tyr Lys
                                    30                                  40
Leu Cys His Pro Glu Glu Leu Val Leu Leu
                                    50

Gly His Ser Leu Gly Ile Pro Trp Ala Pro Leu Ser Ser Cys Pro Ser Gln Ala Leu Gln
                                    60                                  70
Leu Ala Gly Cys Leu Ser Gln Leu His Ser
                                    80

Gly Leu Phe Leu Tyr Gln Gly Leu Leu Gln Ala Leu Glu Gly Ile Ser Pro Glu Leu Gly
                                    90                                  100
Pro Thr Leu Asp Thr Leu Gln Leu Asp Val
                                    110

Ala Asp Phe Ala Thr Thr Ile Trp Gln Gln Met Glu Glu Leu Gly Met Ala Pro Ala Leu
                                    120                                 130
Gln Pro Thr Gln Gly Ala Met Pro Ala Phe
                                    140

Ala Ser Ala Phe Gln Arg Arg Ala Gly Gly Val Leu Val Ala Ser His Leu Gln Ser Phe
                                    150                                 160
Leu Glu Val Ser Tyr Arg Val Leu Arg His
                                    170

Leu Ala Gln Pro
        174
```

## BRIEF DESCRIPTION OF THE DRAWING

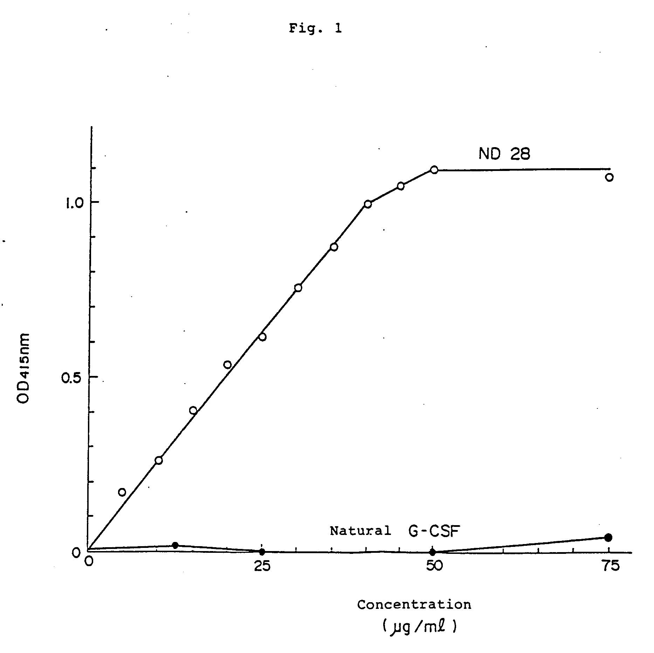

Fig. 1 shows the relationship between the content of ND28 and absorbance at 415 nm, in which ○ represents the data for ND28 and ● the data for natural G-CSF.

DETAILED DESCRIPTION OF THE INVENTION

The monoclonal antibody of this invention can be produced according to the procedure given below.

(1) Preparation of immunized animal cells

Mice (or rats) are immunized with ND28, produced by Escherichia coli based on DNA recombination techniques (Reference Example 1), and the splenic cells are taken out from the immunized animals.

The immunization is carried out by administering ND28 (10 to 100 $\mu$g/head) subcutaneously, intravenously or intraperitoneally to mice of 8- to 10-week age, together with a suitable adjuvant (for example, Complete Freund's Adjuvant, or aluminium hydroxide gel and pertussis vaccine), 2 to 10 times at intervals of one to two weeks; taking blood samples from the venous plexus at the eyeground one week after each administration; and measuring the anti-ND28 antibody titer of the serum by the solid-phase immunoassay as explained below.

The antibody titer is measured by the solid-phase enzyme immunoassay described in "Enzyme Immunoassay" (published from Igaku-shoin in 1978).

100 $\mu\ell$ of a specific antigen solution [a 10 $\mu$g/m$\ell$ ND28 solution in a phosphate-buffered saline (PBS) (solution containing 1.83 g disodium phosphate, 0.21 g monopotassium phosphate and 7.65 g sodium chloride in 1 liter of distilled water; pH 7.2); or a solution of another antigen or BSA in PBS when checking for cross reaction] is dispensed in each well of a 96-well EIA plate (Flow Laboratories, U.S.A.), and the plate is allowed to stand for 12 to 19 hours at 4°C to coat the bottom of each well with the antigen. 1% BSA/PBS solution (200 $\mu\ell$/well) is then dispensed, and the plate is allowed to stand for 12 to 19 hours at 4°C to coat the protein-binding residues on the bottom of each well with BSA. After thorough washing with PBS, serial dilutions of a sample (mouse antiserum, hybridoma culture supernatant, or purified antibody) are dispensed as the first antibody in amount of 50 $\mu\ell$/well and allowed to stand at 4°C for 12 to 19 hours or at 22 to 26°C for three to four hours. After washing six times with PBS, a 1:400 dilution of rabbit antimouse-immunoglobulin/peroxidase conjugate (produced by DAKO and supplied from Kyowa Medex) is then dispensed as the second antibody in amounts of 100 $\mu\ell$/well and allowed to stand at 22 to 26°C for two hours. After washing with PBS, 100 $\mu\ell$ of an ABTS substrate solution [prepared by adding, immediately before use, 1 $\mu\ell$/m$\ell$ hydrogen peroxide to a solution of 550 mg diammonium 2,2'-azino-bis(3-ethylbenzothiazoline-6-sulfonate) in 1 liter of 0.1 M citrate buffer (pH 4.2)] is added to each well, and the color thus developed is measured (at $OD_{415nm}$).

The mice in which the anti-ND28 antibody titer ($OD_{415nm}$) is more than $10^3$ times as high as that of normal mouse serum are used as the suppliers for the immunized animal cells.

ND28 is intraperitoneally administered to these immunized mice (100 $\mu$g/head) three to four days before cell fusion to effect the last immunization, and the spleen is removed from each mouse to prepare the splenic cells to be used for cell fusion.

(2) Preparation of myeloma cells

A mouse-derived cell line is used as the myeloma cells in this invention. Illustrative examples include 8-azaguanine resistant mouse (derived from BALB/c) myeloma cell lines, such as P3-X63 Ag8-Ul (P3-Ul) [Current topics in Microbiology and Immunology, 81, 1-7 (1978)], P3-NSI/1-Ag4.1 (NS-I) [European J. Immunology, 6 511-519 (1976)], SP2/0-Ag14 (SP-2) [Nature, 276, 269-270 (1978)], P3-X63-Ag8.653 (653) [J. Immunology, 123, 1548-1550 (1979)], and P3-X63-Ag8 (X63) [Nature, 256, 495-497 (1975)]. These cell lines, subcultured in 8-azaguanine medium [prepared by adding 1.5 mM glutamine, 5 x $10^{-5}$ M 2-mercaptoethanol, 10 $\mu$g/m$\ell$ gentamycin and 10% fetal calf serum (FCS) to RPMI-1640 medium, and further adding, to the normal medium thus obtained, 15 $\mu$g/m$\ell$ 8-azaguanine], are changed to the normal medium three to four days before cell fusion to secure more than 2 x $10^7$ cells on the day of cell fusion.

(3) Cell fusion

ND28 is intraperitoneally administered (100 $\mu\ell$/head) to the mice immunized in Step (1) above, and the spleen is removed from each mouse three to four days later to prepare splenic cells. These splenic cells and the myeloma cells obtained in Step (2), both thoroughly washed with MEM medium (product of Nissui Seiyaku) or PBS, are mixed together at a cell number ratio of 5~10:1, and the mixture is subjected to centrifugation. After discarding the supernatant, the cell pellet is thoroughly loosened, a mixed solution of 1 to 4 g polyethylene glycol (PEG-1000~4000, product of Wako Pure Chemical Industries), 1 to 4 m$\ell$ MEM

medium and 0.5 to 1.0 mℓ dimethyl sulfoxide was added with stirring in an amount of 0.1 to 1.0 mℓ/$10^8$ - (splenic cell), and the mixture is allowed to stand for 0.5 to 10 minutes. MEM medium (0.5 to 3 mℓ) is then added several times at intervals of 0.5 to 2 minutes, and 30 to 60 mℓ MEM medium is finally added. The resulting mixture is centrifuged, the supernatant is discarded, the cell pellet thus obtained is gently loosened, 50 to 200 ml of the normal medium is added, and the mixture is gently treated with a measuring pipette to suspend the cells. This suspension is dispensed on a culture plate (half the volume of each cell) and incubated at 37°C for 10 to 30 hours in a 3~7%-$CO_2$ incubator. HAT medium (the normal medium supplemented with $10^{-5}$ to $10^{-3}$ M hypoxanthine, $10^{-6}$ to $10^{-4}$ M thymidine and $10^{-8}$ to $10^{-7}$ M aminopterin) is then added to the culture plate (half the volume of each well), and incubation is continued for an additional 10 to 30 hours. After that, half the volume of supernatant in each well is discarded and the same volume of HAT medium is supplied at intervals of 10 to 30 hours over a period of one to three days, and incubation is continued at 37°C for 10 to 14 days.

With the wells in which growth of fused cell colonies is observed, half the volume of supernatant is discarded, the same volume of HT medium (HAT medium with aminopterin removed therefrom) is added, and this medium replacement is repeated at intervals of 10 to 30 hours over a period of one to three days.

After incubation in HT medium for three to four days, a part of culture supernatant is taken out and measured for the anti-ND28 antibody titer by the enzyme immunoassay described above.

With the wells in which an appreciable magnitude of antibody titer is observed, cloning is repeated two to four times by the limiting dilution method, and the cells that show consistent values of antibody titer are selected as a hybridoma cell capable of producing anti-ND28 monoclonal antibody.

(4) Preparation of monoclonal antibody

The monoclonal-antibody producing hybridoma obtained in Step (3) is intraperitoneally administered to pristane-treated BALB/c female mice of 8- to 10-week age (2 to 4 x $10^6$ cell/head). The hybridoma will cause ascites carcinoma in 10 to 21 days. The ascites is taken out of the mice, centrifuged to remove the solid matter, and subjected to ammonium sulfate precipitation (50% and 40% saturation), followed by dialysis against PBS (pH 7.2) for one to two days. The dialysate thus obtained may be submitted to quantitative analysis of ND28 as crude monoclonal antibody.

This crude product can be purified, when required, by passing it through a DEAE-cellulose column or a Protein A-Sepharose column and collecting the IgG fraction.

The isotype and subclass of the monoclonal antibody is determined according to the Ouchterlony method (Biochemical Experiment No.15 (p74) in "A Guide to Experimental Immunology" published from Gakkai Shuppan Center in 1981).

Quantitative analysis of protein is made by Folin's method, the amount being calculated from the absorbance at 280 nm [1.4 ($OD_{280}$) ≒ Immunoglobulin 1 mg/mℓ].

The monoclonal-antibody producing hybridoma of this invention was named KM-498, and the monoclonal antibody KM-498 produced by this hybridoma cell was identified as belonging to the $IgG_1$ isotype.

The antigen specificity of the monoclonal antibody KM-498 is shown in Example 1 given below.

Described below is an example of a ND28 purification procedure using the monoclonal antibody of this invention.

A solution of 10 mg of the monoclonal antibody of this invention in 1 mℓ of PBS is allowed to react with 1 mℓ of CNBr-activated Sepharose-4B (Pharmacia Fine Chemicals) to give immobilized monoclonal antibody. This is charged in a column, and a solution containing ND28 (3 mg or less) prepared by DNA recombination techniques is passed through that column. As a result, 95 to 100% of the ND28 can be adsorbed in the column. Elution with an aqueous solution containing 7M urea and 1M NaCl (pH 7.0) yields about 80% of the adsorbed ND28 as a fraction. Approximately 5000-fold purification can thus be achieved by a single column treatment. In contrast, natural G-CSF is not adsorbed at all in the above column.

The monoclonal antibody of this invention can be used for the quantitative analysis of ND28 by the enzyme immunoassay using a solid-phase sandwich assay technique.

EXAMPLE 1

(1) Preparation of immunized mouse splenic cells

ND28 (100 μg/head) was intraperitoneally administered to five head of BALB/c female mice 8 weeks old (Experimental Animal Agricultural Cooperative Association of Shizuoka Prefecture), together with 2 mg/head of aluminium hydroxide gel and 1 x $10^9$ cell/head of pertussis vaccine (Chiba Serum Research Institute) as

EP 0 331 186 B1

adjuvants to effect the first immunization. After that, 100 $\mu$g/head of ND28 was intraperitoneally administered at intervals of two weeks to effect the second and succeeding immunization. From the third immunization on, blood samples were taken from the venous plexus at the eyeground five to seven days after each administration, and the anti-ND28 antibody titer of the serum was measured by the solid-phase enzyme immunoassay as described above.

An appreciable magnitude of antibody titer was observed with all the five mice after the third immunization, but immunization was repeated five times to effectively obtain a monoclonal antibody of the IgG class.

ND28 (100 $\mu$g/head) was further administered intraperitoneally (final immunization), and the spleen was extracted from each mouse to prepare splenic cells to be used for cell fusion.

(2) Preparation of mouse myeloma cells

Mouse myeloma cells resistant to 8-azaguanine (P3-Ul) were cultured at 37°C in the normal medium (RPMI-1640 medium supplemented with 1.5 mM glutamine, 5 x $10^{-5}$ M 2-mercaptoethanol, 10 $\mu$g/m$\ell$ gentamycin and 0.1 m$\ell$/m$\ell$ FCS), giving more than 2 x $10^7$ cells four days later.

(3) Preparation of hybridoma

The splenic cells of immunized mice (1 x $10^8$) and the mouse myeloma cells P3-Ul (2 x $10^7$), both thoroughly washed with MEM medium (product of Nissui Seiyaku), were mixed together, and the mixture was subjected to centrifugation at 1,200 rpm for five minutes.

The mixture of the two types of cells thus obtained was thoroughly loosened, 0.5 m$\ell$ of a solution prepared by mixing 2 g polyethylene glycol (PEG-1000), 2 m$\ell$ MEM medium and 0.7 m$\ell$ dimethyl sulfoxide was added with stirring at 37°C, and the resulting mixture was allowed to stand for one minute. MEM medium (1 m$\ell$) was then added five times at intervals of one minute, and finally MEM medium was added to make up a total volume of 50 m$\ell$. The resulting mixture was centrifuged at 900 rpm, the supernatant was discarded, the cell pellet thus obtained was gently loosened, 100 m$\ell$ of the normal medium was added, and the mixture was gently treated with a 10-m$\ell$ measuring pipette to suspend the cells.

This suspension was dispensed on a 24-well culture plate (Flow Laboratories, U.S.A.) in amounts of 1 m$\ell$/well and incubated at 37°C for 24 hours in a 5%-$CO_2$ incubator. HAT medium (the above-mentioned normal medium supplemented with $10^{-4}$ M hypoxanthine, 1.5 x $10^{-5}$ M thymidine and 4 x $10^{-7}$ M aminopterin) was then added in amounts of 1 m$\ell$/well, and incubation was continued for 24 hours. After discarding 1 m$\ell$ of supernatant from each well and supplying 1 m$\ell$ of HAT medium, incubation was continued at 37°C for 24 hours. After that, 1 m$\ell$ of culture supernatant was again discarded from each well, 1 m$\ell$ HAT medium was added, and incubation was continued at 37°C for 10 to 14 days.

With the wells in which growth of fused cell colonies was observed, 1 m$\ell$ of culture supernatant was discarded, 1 m$\ell$ of HT medium (HAT medium with aminopterin removed therefrom) was supplied, and incubation was continued at 37°C. This medium replacement was repeated on the following two days, incubation was continued for four days, and a part of culture supernatant was taken out and measured to determine the anti-ND28 antibody titer by the enzyme immunoassay described above.

With the wells in which an appreciable magnitude of antibody titer was observed, cloning was repeated two times by the limiting dilution method, and the cells that showed consistent values of antibody titer were selected as a hybridoma cell capable of producing anti-ND28 monoclonal antibody (hybridoma cell KM-498). This cell was deposited at the Fermentation Research Institute (Agency of Industrial Science and Technology) on January 20, 1988, as murine B cell hybridoma KM-498 FERM BP-1665 in accordance with the Budapest Treaty.

(4) Partial purification of the monoclonal antibody

The hybridoma cell obtained above was intraperitoneally administered to BALB/c female mice 8-weeks of age previously treated with pristane (intraperitoneal administration of 0.5 m$\ell$/head 2,6,10,14-tetramehtylpenta-decane, followed by feeding for two weeks) in an amount of 4 x $10^6$ cell/head. The hybridoma caused ascites carcinoma in 10 to 21 days. The ascites (4 to 10 m$\ell$) was taken out of each mouse, centrifuged to remove the solid matter, and subjected to ammonium sulfate precipitation (50% and 40% saturation), followed by dialysis against PBS (pH 7.2) for two days. The dialyzate thus obtained was recovered as crude monoclonal antibody KM-498.

6

(5) Antigen specificity of the crude monoclonal antibody

The antigen specificity of the crude monoclonal antibody obtained above was measured by the solid-phase enzyme immunoassay. As antigens, there were used ND28, natural G-CSF [Nature, 319, 415 (1986); Science, 232, 61 1986)], foreign proteins derived from host Escherichia coli, and bovine serum albumin (BSA; product of Seikagaku Kogyo Co., Ltd.). The results are shown in Table 2.

| Antibody | Avidity ($OD_{415}$) | | | | |
|---|---|---|---|---|---|
| | concn. or dilution | ND28 | Natural G-CSF | BSA | Foreign proteins derived from E.Coli |
| Normal mouse serum | $\times 10^{-2}$ | 0.020 | 0.015 | 0.010 | 0.060 |
| ND28-Immunized mouse serum | $\times 10^{-2}$ | 0.950 | 0.930 | 0.005 | 0.020 |
| Crude monoclonal antibody KM-498 | 10 $\mu$g/m$\ell$ | 1.280 | 0.005 | 0.005 | 0.025 |

The crude monoclonal antibody was purified by adsorption in a DEAE-Sepharose column, followed by elution and collection of the IgG fraction.

(6) Classification of the monoclonal antibody

As a result of our investigation of the isotype and subclass of the monoclonal antibody KM-498 according to the Ouchterlony method (Biochemical Experiment No.15 (p74) in "A Guide to Experimental Immunology" published from Gakkai Shuppan Center in 1981), it was identified as belonging to the $IgG_1$ class.

EXAMPLE 2

A solution of 10 mg anti-ND28 monoclonal antibody KM-498 obtained in Example 1 in 10 m$\ell$ PBS was allowed to react with 1 m$\ell$ of CNBr-activated Sepharose-4B (Pharmacia Fine Chemicals) to immobilize the monoclonal antibody. This was charged in a column, and 5 m$\ell$ of a culture extract containing 3 mg ND28 was passed through that column. It was found that 2.6 mg (87%) of the ND28 was adsorbed in the column.

After washing with PBS, elution with an aqueous solution containing 7M urea and 1M NaCl gave 2.10 mg ND28 (81% of the adsorbed substance) as a fraction. An approximately 5000-fold purification could be achieved by this single column treatment.

In contrast, natural G-CSF was not adsorbed at all in the above column.

EXAMPLE 3

A 100 $\mu$g/m$\ell$ solution of rabbit antiserum against natural G-CSF (prepared by immunizing rabbits with natural G-CSF) was dispensed as the first antibody on a 96-well EIA plate (Flow Laboratories, U.S.A.) in amounts of 50 $\mu\ell$/well, and allowed to stand at 4°C for 24 hours to coat the bottom surface of each well with this first antibody. A 1% solution of bovine serum albumin in PBS was then dispensed in amounts of 200 m$\ell$/well to cover the rest of protein-binding radicals on the bottom of each well, and allowed to stand at 4°C for 24 hours. After thorough washing with deionized water, 75 to 5 $\mu$g/m$\ell$ solutions of ND28 were dispensed in amounts of 50 $\mu\ell$/well, and the plate was allowed to stand at 22 to 26°C for two hours. After thorough washing with PBS, a 10 $\mu$g/m$\ell$ solution of biotinized KM-498 was dispensed as the second antibody in amounts of 50 $\mu$g/well, and allowed to stand for 12 to 19 hours at 4°C. After washing with PBS, a 10 $\mu$g/m$\ell$ solution of avidin-biotin-peroxidase (product of Vector Inc.) was added in amounts of 100 $\mu\ell$/well, and allowed to stand at 22 to 26°C for one hour. After washing with PBS, an ABTS substrate solution was added in amounts of 100 $\mu\ell$/well, the reaction was allowed to proceed at room temperature for 30 minutes and then terminated by addition of 5% SDS solution (100 $\mu\ell$/well), and the color was measured for each well by the use of an absorptionmeter ($OD_{415}$ nm).

It was demonstrated that, as shown in Fig. 1, ND28 can be quantitatively analyzed in the concentration range of 5 to 50 $\mu$g/m$\ell$. No reaction took place at all when natural G-CSF was used in place of ND28 in this reaction system.

REFERENCE EXAMPLE 1

_Escherichia coli_ ECfBD28 (FERM BP-1479), which carries the recombinant plasmid pcfBD28 that codes for ND28 (EP-A-0272703), was cultured in LG medium (a solution of 10 g Bacto-Tryptone, 5 g yeast extract, 5 g NaCl and 1 g glucose in 1 liter water, with its pH adjusted to 7.0 by addition of caustic soda solution) at 37°C for 18 hours, and 5 mℓ of the culture solution thus obtained was inoculated to 100 mℓ of MCG medium (0.6% $Na_2HPO_4$, 0.3% $KH_2PO_4$, 0.5% NaCl, 0.5% casamino acids, 1 mM $MgSO_4$ and 4 μg/mℓ vitamin $B_1$; pH 7.2) further containing 25 μg/mℓ tryptophan and 50 μg/mℓ ampicillin. The mixture was cultured at 30°C for four to eight hours. Then added was 10 μg/mℓ of 3β-indoleacrylic acid (a tryptophan inducer), and cultivation was continued for an additional 2 to 12 hours. The culture solution was centrifuged for ten minutes at 8,000 rpm, and the collected cells were washed with 30mM Tris-HCl buffer containing 30 mM NaCl (pH 7.5), suspended in 30 mℓ of the same buffer as above and subjected to ultrasonic disruption at 0°C for 10 minutes [treatment for ten minutes in Sonifier Cell Disruptor 200, output control 2 (Branson Sonic Power Company)]. The cell debris thus obtained was centrifuged for 30 minutes at 9,000 rpm, and ND28 was extracted from the residue and purified by the method of A.O. Marston et al. [BIO/TECHNOLOGY, 2, 800 (1984)].

**Claims**

1. A monoclonal antibody of the $IgG_1$ isotype that selectively binds to a protein having the amino acid sequence 1-174 as shown in Table 1, which is a derivative of human granulocyte colony stimulating factor, but does not bind to natural human granulocyte colony stimulating factor.

2. The monoclonal antibody of claim 1 which is secreted by hybridoma cell line FERM Deposit No. BP-1665 and its progeny.

3. A monoclonal antibody from hybridoma cell line Deposit No. FERM BP-1665 and its progeny

4. A process for purifying a protein having the amino acid sequence 1-174 as shown in Table 1 comprising passing a solution containing the protein to be purified through an affinity chromatography column filled with a monoclonal antibody of $IgG_1$ isotype that binds to the protein to be purified but does not bind to natural human granulocyte colony stimulating factor and is immobilized on an inert support, adsorbing the protein to be purified onto the immobilized antibody, and eluting the adsorbed protein and recovering same in substantially pure form.

5. The process of claim 4 in which the solution containing the protein to be purified also contains natural human granulocyte colony stimulating factor.

6. The process of claim 5 in which the protein to be purified is recovered in substantially pure form substantially completely devoid of natural human granulocyte colony stimulating factor.

7. The process of claim 4 in which the monoclonal antibody is secreted from hybridoma cell line Deposit No. FERM BP-1665 and its progeny.

8. The process of claim 5 in which the monoclonal antibody is secreted from hybridoma cell line Deposit No. FERM BP-1665 and its progeny.

9. A method for quantitatively determining the amount of a protein having the amino acid sequence 1-174 as shown in Table 1 in a sample comprising subjecting the sample to enzyme solid-phase sandwich immunoassay in which an antibody against natural human granulocyte colony stimulating factor is used as a first antibody and a labelled monoclonal antibody that selectively binds to the protein to be quantified but not to natural human granulocyte colony stimulating factor is used, reacting the labelled antibody with an enzyme and assessing the sample for the relative quantification of the protein of interest.

**Patentansprüche**

1. Monoklonaler Antikörper des Isotyps $IgG_1$, der selektiv an ein Protein bindet, welches die in Tabelle 1 gezeigte Aminosäuresequenz 1-174 aufweist, das ein Derivat des menschlichen Granulozyten-Kolonie-stimulierenden Faktors ist, der jedoch nicht an natürlichen, menschlichen Granulozyten-Kolonie-stimulierenden Faktor bindet.

2. Monoklonaler Antikörper nach Anspruch 1, der von der Hybridomzellinie FERM Hinterlegungs-Nr. BP-1665 und deren Nachkommen ausgeschieden wird.

3. Monoklonaler Antikörper aus der Hybridomzellinie mit der Hinterlegungs-Nr. FERM BP-1665 und deren Nachkommen.

4. Verfahren zur Reinigung eines Proteins mit der in der Tabelle 1 gezeigten Aminosäuresequenz 1-174, wobei man eine Lösung, die das zu reinigende Protein enthält, über eine Affinitäts-Chromatographiesäule leitet, welche mit einem monoklonalen Antikörper des Isotyps $IgG_1$ gefüllt ist, der an das zu reinigende Protein bindet, jedoch nicht an natürlichen, menschlichen Granulozyten-Kolonie-stimulierenden Faktor bindet, und auf einem inerten Träger immobilisiert ist, das zu reinigende Protein an dem immobilisierten Antikörper adsorbiert, und das adsorbierte Protein eluiert und dieses in im wesentlichen reiner Form isoliert.

5. Verfahren nach Anspruch 4, wobei die das zu reinigende Protein enthaltende Lösung zusätzlich natürlichen, menschlichen Granulozyten-Kolonie-stimulierenden Faktor enthält.

6. Verfahren nach Anspruch 5, wobei das zu reinigende Protein in im wesentlichen reiner Form isoliert wird, die im wesentlichen vollständig frei ist von natürlichem, menschlichen Granulozyten-Kolonie-stimulierendem Faktor.

7. Verfahren nach Anspruch 4, wobei der monoklonale Antikörper von der Hybridomzellinie mit der Hinterlegungs-Nr. FERM BP-1665 und deren Nachkommen ausgeschieden wird.

8. Verfahren nach Anspruch 5, wobei der monoklonale Antikörper von der Hybridomzellinie mit der Hinterlegungs-Nr. FERM BP-1665 und deren Nachkommen ausgeschieden wird.

9. Verfahren zur quantitativen Bestimmung der Menge eines Proteins mit der in der Tabelle 1 gezeigten Aminosäuresequenz 1-174 in einer Probe, wobei man die Probe einem enzymatischen Festphasen-Sandwich-Immunoassay unterzieht, bei dem ein Antikörper gegen natürlichen, menschlichen Granulozyten-Kolonie-stimulierenden Faktor als erster Antikörper verwendet wird und ein markierter monoklonaler Antikörper, der selektiv an das zu quantifizierende Protein bindet, jedoch nicht an natürlichen, menschlichen Granulozyten-Kolonie-stimulierenden Faktor bindet, verwendet wird, man den markierten Antikörper mit einem Enzym umsetzt und die relative Quantifizierung des interessierenden Proteins in der Probe durchführt.

**Revendications**

1. Anticorps monoclonal d'isotype $IgG_1$ qui se fixe sélectivement sur une protéine ayant la séquence d'acides aminés 1-174 présentée dans le tableau 1, qui est un dérivé du facteur de stimulation des colonies de granulocytes humain naturel, mais qui ne se fixe pas sur le facteur de stimulation des colonies de granulocytes humain naturel.

2. Anticorps monoclonal de la revendication 1, qui est sécrété par la lignée cellulaire d'hybridome No. de dépôt FERM BP-1665 et ses descendants.

3. Anticorps monoclonal provenant de la lignée cellulaire d'hybridome No. de dépôt FERM BP-1665 et ses descendants.

4. Procédé de purification d'une protéine ayant la séquence d'acides aminés 1-174 présentée dans le tableau 1, comprenant le passage d'une solution contenant la protéine à purifier sur une colonne de chromatographie d'affinité remplie par un anticorps monoclonal d'isotype IgG$_1$ qui se fixe sur la protéine à purifier mais ne se fixe pas sur le facteur de stimulation des colonies de granulocytes humain naturel et est immobilisé sur un support inerte, l'adsorption de la protéine à purifier sur l'anticorps immobilisé, et l'élution de la protéine adsorbée et la récupération de celle-ci sous forme pratiquement pure.

5. Procédé de la revendication 4, dans lequel la solution contenant la protéine à purifier contient également le facteur de stimulation des colonies de granulocytes humain naturel.

6. Procédé de la revendication 5, dans lequel la protéine à purifier est récupérée sous forme pratiquement pure pratiquement complètement dépourvue du facteur de stimulation des colonies de granulocytes humain naturel.

7. Procédé de la revendication 4, dans lequel l'anticorps monoclonal est sécrété à partir de la lignée cellulaire d'hybridome No. de dépôt FERM BP-1665 et ses descendants.

8. Procédé de la revendication 5, dans lequel l'anticorps monoclonal est sécrété à partir de la lignée cellulaire d'hybridome No. de dépôt FERM BP-1665 et ses descendants.

9. Procédé de détermination quantitative de la quantité d'une protéine ayant la séquence d'acides aminés 1-174 présentée dans le tableau 1 dans un échantillon, comprenant la soumission de l'échantillon à un immunodosage enzymatique en sandwich en phase solide, dans lequel un anticorps contre le facteur de stimulation des colonies de granulocytes humain naturel est utilisé comme un premier anticorps, et un anticorps monoclonal marqué qui se fixe sélectivement sur la protéine à quantifier mais pas sur le facteur de stimulation des colonies de granulocytes humain naturel est utilisé, la réaction avec l'anticorps marqué avec une enzyme et l'évaluation de l'échantillon en ce qui concerne la quantification relative de la protéine intéressante.

Fig. 1